# FASCICULE DE BREVET EUROPEEN

(11) **EP 4 062 922 B1**
(45) Date de publication et mention de la délivrance du brevet: **10.01.2024**
(21) Numéro de dépôt: 21305353.1
(22) Date de dépôt: 22.03.2021
(51) Int. Cl.: A61K 36/51, A61P 17/00, A61K 8/9789, A61Q 19/00, A61Q 19/08

(54) **EXTRAIT DE SWERTIA CHIRATA ET COMPOSITIONS COSMÉTIQUES LE COMPRENANT**
EXTRAKT AUS SWERTIA CHIRATA UND KOSMETISCHE ZUSAMMENSETZUNGEN, DIE DIESE ENTHALTEN
EXTRACT OF SWERTIA CHIRATA AND COSMETIC COMPOSITIONS COMPRISING SAME

(43) Date de publication de la demande: 28.09.2022
(73) Titulaire: Chanel Parfums Beauté, 92200 Neuilly-sur-Seine (FR)
(72) Inventeur: RILHAC, Vincent, 93694 PANTIN CEDEX (FR); GILLET, Maeva, 93694 PANTIN CEDEX (FR)
(74) Mandataire: Plasseraud IP

(56) Documents cités:
- EP-B1- 1 928 447
- US-A1- 2017 020 796
- Gnpd Mintel: "Restorative Cream Product Details Company Indeed Laboratories, Canada Brand Indeed Laboratories Me-NO-Pause Category Skincare > Face/Neck Care Market UK IMPORTED PRODUCT Company & Source Details Store Name FeelUnique Store Type Internet/Mail Order Product Description", , 1 novembre 2020 (2020-11-01), XP055850054, Extrait de l'Internet: URL:https://www.gnpd.com/sinatra/recordpag e/8192211/ [extrait le 2021-10-11]
- KAUR PRABHJOT ET AL: "Simultaneous quantification of oleanolic acid, ursolic acid, betulinic acid and lupeol in different populations of fiveSwertiaspecies by using HPTLC-densitometry: Comparison of different extraction methods and solvent selection", INDUSTRIAL CROPS AND PRODUCTS, vol. 130, 2019, pages 537-546, XP085588838, ISSN: 0926-6690, DOI: 10.1016/J.INDCROP.2018.12.089
- VIJAY KUMAR ET AL: "A Review of Swertia chirayita (Gentianaceae) as a Traditional Medicinal Plant", FRONTIERS IN PHARMACOLOGY, vol. 6, 12 janvier 2016 (2016-01-12), pages 1-14, XP055467762, DOI: 10.3389/fphar.2015.00308

## Description

Chanel Parfums Beauté souhaite remercier le Centre National de Biodiversity du Bhoutan pour son accompagnement dans la mise en place de la filière d'approvisionnement et la mise en culture de la plante *Swertia chirata* ainsi que dans la mise en application du Protocole de Nagoya.

Nos Sincères remerciements au Secrétaire Général Mr Dasho Rinzin Dorji, à la Directrice de Programme Dr. Tashi Yangzome Dorji et à l'ensemble de l'équipe Bioprospection : Mr Chencho Dorji, Mr Mani Prasad Nirola, Mrs Jamyang Choden et tout particulièrement Mr Leki Wangchuk, pour leur soutien et conseils.

### Domaine de l'invention

La présente invention a pour objet un extrait de *Swertia chirata,* son procédé d'obtention, une composition cosmétique le comprenant, ainsi que différentes utilisations cosmétiques.

### Arrière-plan technique de l'invention

La peau est principalement constituée de trois couches, à savoir, en partant de la plus superficielle, l'épiderme, le derme et l'hypoderme.
La couche externe de la peau, l'épiderme, est constituée de kératinocytes (majoritaires), de mélanocytes (intervenant dans la pigmentation de la peau) et de cellules de Langerhans. Sa fonction est de protéger le corps de l'environnement extérieur et d'assurer son intégrité, et notamment de freiner la pénétration de micro-organismes ou de substances chimiques, et d'empêcher l'évaporation de l'eau contenue dans la peau.

Pour ce faire, les kératinocytes subissent un processus de maturation orientée continu au cours duquel les kératinocytes situés dans la couche basale de l'épiderme forment, au stade terminal de leur différenciation, des cornéocytes qui sont des cellules mortes totalement kératinisées sous forme d'enveloppes cornées constituées de protéines et de lipides tels que des céramides. Lors de ce processus de différenciation, des lipides épidermiques intercornéocytaires sont en outre formés puis organisés sous forme de bicouches (feuillets) dans le stratum corneum. Ils participent, avec les enveloppes cornées précitées, à la fonction barrière de l'épiderme.

Lors du vieillissement cutané, la peau s'affine, elle perd en volume et élasticité et sa fonction barrière s'en trouve altérée. La jonction dermo-épidermique est une des cibles privilégiées du vieillissement cutané. Sa fonction principale est de permettre la cohésion entre l'épiderme et le derme afin d'assurer les fonctions de cohésion et de nutrition de la peau. Ces fonctions reposent principalement sur l'action de la matrice extracellulaire qui est produite par les kératinocytes. Cette matrice est constituée d'un assemblage de plusieurs macromolécules dont le collagène, des protéoglycanes, des glycosaminoglycanes (dont l'acide hyaluronique), de l'élastine et des glycoprotéines de structure. Le vieillissement cellulaire et l'action de facteurs environnementaux tels que les UV ou la pollution vont altérer ces composants de la matrice extracellulaire. Ils vont subir une dégradation enzymatique, notamment via l'action des métalloprotéinases matricielles (MMP), la matrice n'arrivera plus à assurer ses fonctions proprement, ce qui va engendrer des fragilités et un abaissement de la capacité de régénération cellulaire et être l'origine de l'apparition rides et ridules sur la peau.

La matrice extracellulaire et la prévention de sa dégradation, résultant notamment de l'action d'enzymes telles que les MMP, sont devenues des cibles privilégiées dans la prévention et la réduction du vieillissement cutané.

De ce fait, il existe un réel besoin de fournir des agents actifs susceptibles d'agir sur le maintien des fonctions de la matrice extracellulaire et notamment sur la prévention de sa dégradation pour lutter contre les altérations dues au vieillissement cutané.

De plus, en raison d'une volonté toujours plus grande des consommateurs de se tourner vers des produits naturels renfermant le moins d'ingrédients synthétiques possibles, et au vu des contraintes réglementaires de plus en plus lourdes pesant sur les composés issus de l'industrie chimique, il serait avantageux que de tels actifs soient issus d'extraits végétaux.

Il est aussi nécessaire de rechercher des procédés d'extraction plus respectueux de l'environnement en utilisant des solvants recyclables et non toxiques tout en permettant une extraction sélective des molécules d'intérêt sans les dégrader

US2017/020796 divulgue l'utilisation cosmétique de la swertiamarine ou d'un extrait végétal enrichi en swertiamarine.

Kaur Prabhjot et al. divulgue des méthodes d'extraction pour Swertia.

### Résumé de l'invention

*Swertia chirata* est une plante du genre *Swertia,* un genre appartenant à la famille des *Gentianaceae* et qui regroupe environ 150 espèces. *Swertia chirata* est notamment utilisée dans la médecine traditionnelle en Inde, au Bhoutan et au Népal, d'où elle est originaire. Ses racines, qui sont réputées pour être la partie la plus bioactive de la plante, sont utilisées pour traiter diverses affections comme la fièvre, le paludisme, l'asthme, certaines pathologies du foie, le diabète ou encore certains types de troubles mentaux.

De manière tout à fait surprenante, les auteurs de la présente invention ont réussi à démontrer qu'il était possible d'obtenir des extraits de *Swertia chirata* ayant des propriétés antiâges et protectrices de la fonction barrière remarquables en faisant subir une étape d'extraction au CO₂ supercritique à un extrait alcoolique de *Swertia chirata.* Les inventeurs ont notamment démontré que ces extraits permettaient d'un côté d'inhiber le processus d'inflammation et de dégradation de la matrice extracellulaire médiés par les kératinocytes épidermiques, et d'un autre côté de stimuler le métabolisme lipidique des kératinocytes et de stimuler la synthèse des composants de la matrice extracellulaire. Ces extraits peuvent donc être utilisés pour prévenir/ralentir le vieillissement cutané résultant de la désorganisation et de la dégradation de la matrice extracellulaire, mais aussi pour améliorer la fonction barrière et l'hydratation de la peau.

Les extraits de *Swertia chirata* selon l'invention sont, de plus, avantageusement compatibles avec toutes les formes galéniques : non seulement ils peuvent être utilisés dans des compositions cosmétiques telles que des crèmes, des gel-crèmes, mais ils peuvent également être avantageusement introduits dans des compositions ne comprenant qu'une phase aqueuse telles que des lotions, ou dans des compositions ne comprenant qu'une phase non-aqueuse, par exemple sous forme d'huiles.

Aussi, selon un premier aspect, la présente invention porte sur un procédé de préparation d'un extrait de *Swertia chirata,* comprenant une étape d'extraction au CO₂ supercritique d'un extrait alcoolique de *Swertia chirata,* à une température comprise entre 40 et 80°C, de préférence entre 50 et 70°C et à une pression comprise entre 20 et 60 MPa, de préférence entre 25 et 35 MPa.

L'invention a également pour objet un extrait de *Swertia chirata* obtenu à partir d'un tel procédé, ainsi qu'une composition cosmétique comprenant, dans un milieu physiologiquement acceptable, un tel extrait de *Swertia chirata.*

Enfin, l'invention a également pour objet, l'utilisation cosmétique non thérapeutique d'un extrait de *Swertia chirata* tel que décrit précédemment, pour prévenir et/ou réduire le vieillissement cutané mais aussi pour hydrater la peau et/ou améliorer la fonction barrière de la peau.

### Description Détaillée

### Swertia chirata

*Swertia chirata,* également appelée *Swertia chirayta, Swertia chirayita* ou encore chirette, est une herbacée appartenant à la famille des *Gentianaceae* et au genre *Swertia.* On la trouve sur les plateaux himalayens, notamment en Inde, au Népal et au Bhoutan. C'est une herbacée annuelle/bisannuelle érigée de 60 à 150cm de haut, ayant une tige cylindrique de sa base jusqu'à son milieu, et quadrangulaire sur sa partie supérieure. Sa tige est brun-orangée, voire violacée, avec un coeur jaune. Ses feuilles sont lancéolées, opposées, sessiles, acuminées et d'environ 4 à 10cm de long. Ses fleurs sont nombreuses, tétramériques et d'un vert-jaunâtre teinté de violet.

L'extrait de *Swertia chirata* selon l'invention est obtenu partir de la plante entière, c'est-à-dire des parties comprenant les fleurs, les feuilles, la tige et les racines de la plante.

### Procédé de préparation d'un extrait de Swertia chirata

Le procédé de préparation d'un extrait de *Swertia chirata* selon la présente invention comprend une étape d'extraction au CO₂ supercritique d'un extrait alcoolique de *Swertia chirata,* à une température comprise entre 40 et 80°C, de préférence entre 50 et 70°C et à une pression comprise entre 20 et 60 MPa, de préférence entre 25 et 35 MPa.

La demanderesse a en effet démontré qu'en procédant à une seconde extraction d'un extrait alcoolique de *Swertia chirata* au moyen de CO₂ supercritique, il était possible d'obtenir des extraits ayant des propriétés antiâges et stimulatrices de la fonction barrière remarquables.

L'extraction au CO₂ supercritique est opérée à une température comprise entre 40 et 80°C, de préférence entre 50 et 70°C, par exemple à 60°C, et à une pression comprise entre 20 et 60 MPa, de préférence entre 25 et 35 MPa, par exemple 30 MPa.

Dans le cadre de la présente invention, il est à noter qu'un intervalle de valeur compris entre « A » et « B », comme la température ci-dessus qui est comprise entre 40 et 80°C, comprend toutes les valeurs comprises entre A et B, bornes incluses. L'étape d'extraction au CO₂ supercritique selon la présente invention peut donc être opérée à n'importe quelle valeur de température comprise entre 40 et 80°C, mais aussi à 40 ou 80°C. Ceci s'applique pour toutes les plages de valeurs divulguées dans le cadre de la présente invention.

Selon un mode préféré de réalisation, le débit de CO₂ est d'au moins 150g/min, de préférence 200g/min.

L'extraction au CO₂ supercritique est préférentiellement opérée en présence d'un solvant alcoolique. Le solvant alcoolique peut être un monoalcool comprenant de 2 à 4 atomes de carbone, préférentiellement l'éthanol. Selon un mode de réalisation préféré, l'extraction au CO₂ supercritique est opérée en présence d'éthanol, préférentiellement de l'éthanol à 60°.

Selon un mode de réalisation particulier, le solvant alcoolique, de préférence l'éthanol, est introduit selon une teneur comprise entre 1 et 10% par rapport au poids total du mélange comprenant le CO₂ supercritique et le solvant alcoolique.

L'extraction par CO₂ supercritique a pour avantages de ne nécessiter qu'une faible consommation en solvants (le CO₂ supercritique, solvant majoritaire, est recyclé), d'utiliser des solvants non toxiques et d'être un procédé respectueux de l'environnement.

Les solvants d'extraction (mélange comprenant le CO₂ supercritique et optionnellement le solvant alcoolique) sont mis en oeuvre dans l'étape d'extraction au CO₂ supercritique en un ratio massique des solvants d'extraction par rapport à l'extrait alcoolique compris entre 40 et 150, préférentiellement 75.

Dans le cadre de la présente invention, l'« extrait alcoolique » qui subit l'étape d'extraction au CO₂ supercritique est un extrait qui a été obtenu par une étape d'extraction de *Swertia chirata* avec au moins un solvant alcoolique, et qui a donc subit une étape d' « extraction alcoolique ». Le solvant alcoolique est tel que défini ci-dessus. Il peut être le même ou un autre solvant alcoolique que celui utilisé pour l'étape d'extraction au CO₂ supercritique. Selon un mode de réalisation préféré, le solvant alcoolique utilisé pour l'étape d'extraction alcoolique est l'éthanol, plus préférentiellement de l'éthanol à 60°.

Toutes les parties de *Swertia chirata* peuvent être extraites avec le solvant alcoolique sous forme « fraîche », c'est-à-dire dans les 48 heures, particulièrement les 24 heures, encore plus particulièrement les 12 heures suivant la récolte.

Toutes les parties de *Swertia chirata* peuvent également être extraites sous forme sèche. Dans ce cas, la plante fraîche est déshydratée en conditions douces, soit à température ambiante à l'abri de la lumière soit dans un séchoir ventilé à une température inférieure à 35°C. La plante est de préférence séchée jusqu'à obtention d'une teneur en matière sèche supérieure à 80% et préférentiellement supérieure à 85%.

Toutes les parties de *Swertia chirata* peuvent en outre être broyées afin d'obtenir une poudre comprenant des particules de taille inférieure à 5 cm, de préférence inférieure à 2 cm.

Toutes les parties de *Swertia chirata,* sous forme fraîche ou sous forme sèche, entières ou broyées, sont soumises à au moins une étape d'extraction avec au moins un solvant alcoolique (étape d'extraction alcoolique) pour obtenir l'extrait alcoolique de *Swertia chirata* utilisé dans le cadre de la présente invention. Le ratio plante/solvant alcoolique pour cette étape d'extraction alcoolique est typiquement de 1 pour 10 (poids/poids), et cette étape dure au moins 1 heure, de préférence au moins 2 heures, particulièrement au moins 3 heures, et peut être renouvelée une à deux fois. L'extrait alcoolique obtenu à l'issue de cette étape d'extraction alcoolique est filtré, par exemple par tamisage, afin d'éliminer les résidus végétaux. L'extrait alcoolique peut, par exemple, être filtré à 100µm, particulièrement 10µm, préférentiellement 1µm.

L'extrait alcoolique peut en outre être décoloré, par exemple après l'étape de filtration. Cette étape vise à éliminer les pigments présents dans l'extrait (majoritairement de la chlorophylle). L'homme du métier connaît plusieurs méthodes permettant d'éliminer les pigments présents dans les extraits de plantes. La décoloration peut, par exemple, être effectuée en mettant l'extrait en contact avec du charbon actif. Une fois la décoloration effectuée, le mélange est filtré afin d'éliminer les résidus de charbon.

C'est donc cet extrait alcoolique de *Swertia chirata,* obtenu après au moins une étape d'extraction alcoolique de *Swertia chirata,* et éventuellement une étape de filtration et/ou de décoloration, qui va être soumis à l'extraction au CO₂ supercritique.

Dans un mode de réalisation particulier, l'extrait alcoolique est associé à un support tel qu'un polysaccharide comme de l'amidon, de la maltodextrine, ou de préférence de la cellulose, avant d'être soumis à l'extraction au CO₂ supercritique. Dans ce cadre, le polysaccharide est ajoutée à l'extrait alcoolique : la teneur en extrait alcoolique va de 50 à 70% en poids par rapport au poids total du mélange comprenant l'extrait alcoolique et le polysaccharide, et la teneur en polysaccharide (de préférence de la cellulose) va de 30 à 50% en poids par rapport au poids total du mélange comprenant l'extrait alcoolique et le polysaccharide.

Selon ce mode de réalisation particulier, le solvant alcoolique, préférentiellement l'éthanol, présent dans le mélange extrait alcoolique + polysaccharide peut être éliminé, typiquement par évaporation, par exemple par évaporation sous vide, afin d'obtenir uniquement un mélange polysaccharide/extrait sec, c'est à dire comprenant 100% en poids de matière sèche. Ce mélange peut ensuite être broyé afin d'obtenir une poudre polysaccharide/extrait sec comprenant des particules de taille inférieure à 2 cm, de préférence inférieure à 1 cm. Selon ce mode de réalisation, c'est cette poudre qui est soumise à l'étape d'extraction au CO₂ supercritique dans le cadre du procédé de la présente invention.

Aussi, selon un mode de réalisation particulier, le procédé de préparation d'un extrait de *Swertia chirata* selon la présente invention comprend les étapes suivantes :
a) l'extraction de *Swertia chirata,* de préférence sous forme de poudre, avec au moins un solvant alcoolique, de préférence de l'éthanol ;
b) la filtration du mélange obtenu en a) afin d'éliminer les résidus végétaux ;
b') optionnellement, la décoloration du mélange obtenu à l'issue de l'étape b) ;
c) l'ajout d'un polysaccharide, de préférence de la cellulose, dans le mélange ainsi obtenu afin d'obtenir un mélange polysaccharide/extrait sec dans le solvant alcoolique ;
d) l'élimination du solvant alcoolique, de préférence par évaporation ;
e) le broyage du mélange extrait sec/polysaccharide obtenu à l'issue de l'étape d) afin d'obtenir une poudre ; et
f) l'extraction au CO₂ supercritique de la poudre obtenue à l'issue de l'étape e) à une température comprise entre 40 et 80°C, de préférence entre 50 et 70°C et à une pression comprise entre 20 et 60 MPa, de préférence entre 25 et 35 MPa.

L'extrait de *Swertia chirata* obtenu à l'issue de l'étape d'extraction au CO₂ supercritique est l'extrait selon l'invention.

Cet extrait peut en outre être traité et finalisé à des fins cosmétiques, afin de pouvoir être intégré dans une composition cosmétique. Ce traitement est effectué par des moyens classiques, connus de l'homme du métier, par exemple via des étapes de dilution, d'association à un support cosmétique, d'évaporation des solvants ou encore de filtration.

Dans ce cadre, l'extrait contenu dans le solvant utilisé pour l'extraction au CO₂ supercritique (c'est-à-dire la phase liquide obtenue à l'issue de l'étape d'extraction au CO₂ supercritique) peut subir une étape de filtration, par exemple jusqu'à 1µm.

L'extrait peut ensuite être dilué dans une huile telle que des huiles végétales ou des huiles estérifiées, comme du caprylic/capric triglycéride ou de préférence du palmitate ethyl-2-hexyl, par exemple à 1%.

Le solvant alcoolique peut être éliminé avant ou après dilution dans l'huile, par exemple par évaporation, de manière préférée par évaporation sous vide.

L'extrait dilué dans l'huile peut ensuite être à nouveau filtré, par exemple jusqu'à 1µm afin d'éliminer les insolubles et ainsi obtenir l'extrait final.

Selon ce mode de réalisation, le procédé ci-dessus peut donc comprendre en outre les étapes g) à i) suivantes :
g) la filtration de l'extrait contenu dans le solvant utilisé pour l'extraction au CO₂ supercritique, et éventuellement l'élimination du solvant alcoolique, de préférence par évaporation ;
h) la dilution de l'extrait obtenu à l'issue de l'étape g) dans une huile, de préférence du palmitate ethyl-2-hexyl, et éventuellement l'élimination du solvant alcoolique résiduel, de préférence par évaporation ;
i) la filtration de l'extrait dilué obtenu à l'issue de l'étape h).

Selon ce mode de réalisation, le procédé de préparation d'un extrait de *Swertia chirata* selon la présente invention peut donc comprendre les étapes suivantes :
a) Toutes les parties de Swertia chirata préalablement séchées et broyées à une finesse inférieure à 2cm sont extraites 2 fois par macération dans l'éthanol à 60°C pendant 2h avec un rapport solvant/plante de 10/1 ;
b) L'extrait obtenu est ensuite tamisé à 100µm puis filtré jusqu'à 1µm pour éliminer les résidus végétaux ;
b') L'extrait est décoloré avec 30% de charbon actif par rapport à l'extrait sec et ensuite à nouveau filtré à 1µm pour éliminer le charbon résiduel ;
c) De la cellulose est ajoutée à l'extrait pour obtenir un mélange cellulose (40%) / extrait sec (60%) dans l'éthanol ;
d) L'éthanol est ensuite évaporé pour obtenir uniquement le mélange cellulose/extrait sec ;
e) Le mélange cellulose/extrait sec est ensuite broyé à une finesse inférieure à 1cm pour obtenir une poudre ;
f) La poudre obtenue est ensuite extraite par un mélange CO₂ supercritique (95%) / Ethanol (5%) à 60°C, à 500 bars (50MPa) et avec un débit de 200g/min jusqu'à ce que le ratio massique du mélange CO₂ supercritique (95%) / Ethanol (5%) par rapport à l'extrait alcoolique soit de 75;
g) L'extrait obtenu contenu dans l'éthanol est filtré à 1µm ;
h) L'extrait obtenu à l'issue de l'étape g) est dilué à 1% dans le palmitate ethyl-2-hexyl et l'éthanol est évaporé ;
i) L'extrait est ensuite filtré à 1µm pour éliminer les insolubles et obtenir l'extrait final (nommé ici « extrait C1 »).

Selon un mode de réalisation alternatif, la poudre résiduelle, qui contient le polysaccharide tel que la cellulose et une partie de l'extrait de *Swertia chirata,* obtenue à l'issue de l'étape d'extraction au CO₂ supercritique (c'est-à-dire la phase solide obtenue à l'issue de l'étape d'extraction au CO₂ supercritique) peut être mise en suspension dans un solvant alcoolique, de préférence l'éthanol. Le solvant alcoolique est défini comme précédemment et peut être le même ou un autre solvant alcoolique que celui utilisé pour l'étape d'extraction alcoolique et/ou pour l'étape d'extraction au CO₂ supercritique.

Le mélange contenant la poudre résiduelle en suspension dans le solvant alcoolique peut ensuite être filtré, par exemple jusqu'à 1µm, afin d'éliminer la cellulose.

L'extrait peut ensuite être dilué dans un glycol tel que du caprylyl glycol, du pentylene glycol, ou de préférence du propanediol, par exemple à 1%.

Le solvant alcoolique peut être éliminé avant ou après dilution dans le glycol, par exemple par évaporation, de manière préférée par évaporation sous vide.

L'extrait dilué dans le glycol peut ensuite être à nouveau filtré, par exemple jusqu'à 1µm afin d'éliminer les insolubles et obtenir ainsi l'extrait final

Selon ce mode de réalisation alternatif, le procédé comprenant les étapes a) à f) ci-dessus peut donc comprendre en outre les étapes g1) à j1) suivantes :
g1) mise en solution de la poudre résiduelle obtenue à l'issue de l'étape f) dans un solvant alcoolique, de préférence l'éthanol ;
h1) filtration du mélange obtenu à l'issue de l'étape g1) et éventuellement l'élimination du solvant alcoolique, de préférence par évaporation ;
i1) dilution de l'extrait obtenu à l'issue de l'étape h1) dans un glycol, de préférence du propanediol, et éventuellement l'élimination du solvant alcoolique résiduel, de préférence par évaporation ;
j1) filtration de l'extrait dilué obtenu à l'issue de l'étape i1).

Selon ce mode de réalisation alternatif, le procédé de préparation d'un extrait de *Swertia chirata* selon la présente invention peut donc comprendre les étapes suivantes :
a) Toutes les parties de Swertia chirata préalablement séchées et broyées à une finesse inférieure à 2cm sont extraites 2 fois par macération dans l'éthanol à 60°C pendant 2h avec un rapport solvant/plante de 10/1 ;
b) L'extrait obtenu est ensuite tamisé à 100µm puis filtré jusqu'à 1µm pour éliminer les résidus végétaux ;
b') L'extrait est décoloré avec 30% de charbon actif par rapport à l'extrait sec et ensuite à nouveau filtré à 1µm pour éliminer le charbon résiduel ;
c) De la cellulose est ajoutée à l'extrait pour obtenir un mélange cellulose (40%) / extrait sec (60%) dans l'éthanol ;
d) L'éthanol est ensuite évaporé pour obtenir uniquement le mélange cellulose/extrait sec ;
e) Le mélange cellulose/extrait sec est ensuite broyé à une finesse inférieure à 1cm pour obtenir une poudre ;
f) La poudre obtenue est ensuite extraite par un mélange CO₂ supercritique (95%) / Ethanol (5%) à 60°C, à 500 bars (50MPa) et avec un débit de 200g/min jusqu'à ce que le ratio massique du mélange CO₂ supercritique (95%) / Ethanol (5%) par rapport à l'extrait alcoolique soit de 75;
g1) La poudre résiduelle présente à l'issue de l'étape f) est alors récupérée et remise en solution dans l'éthanol sous agitation à 45°C ;
h1) Le mélange est alors filtré à 1µm pour éliminer la cellulose ;
i1) L'extrait est alors dilué à 1% dans le propanediol et l'éthanol est évaporé sous vide;
j1) L'extrait est ensuite filtré à 1µm pour éliminer les insolubles et obtenir l'extrait final (nommé ici « extrait C2 »).

### Extrait de Swertia chirata

L'invention objet de la présente demande couvre également un extrait de *Swertia chirata* obtenu au moyen du procédé précédemment décrit.

### Composition cosmétique

La présente invention a encore pour objet une composition cosmétique comprenant, dans un milieu physiologiquement acceptable, au moins un extrait de *Swertia chirata* obtenu selon le procédé de la présente invention.

La composition mise en oeuvre selon l'invention comprend généralement, outre l'extrait décrit précédemment, un milieu physiologiquement acceptable et de préférence cosmétiquement acceptable, c'est-à-dire qui convient à une utilisation en contact avec la peau humaine sans risque de toxicité, d'incompatibilité, d'instabilité, de réponse allergique et notamment qui ne provoque pas de sensations d'inconfort (rougeurs, tiraillements, picotements).

Avantageusement, ladite composition cosmétique ou dermatologique peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau dans huile ou huile dans eau.

La composition cosmétique ou dermatologique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- Un ou plusieurs agent(s) humectants, tels que les polyols (glycérine, diglycérine, le propanediol, le caprylyl glycol, le pentylène glycol, l'hexanediol), les sucres, les glycosaminoglycanes tels que l'acide hyaluronique et ses sels et esters; et les polyquaterniums tel que le lipidure PMB
   Ledit agent humectant sera présent dans la composition à une teneur de l'ordre de 0.1 à 30%, de préférence 0.005 à 10% en poids total de la composition.
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglycéride), les beurres tels que les beurres de karité (butyrospernum parkii butter extract, shea butter ethyl esters, commercialisés sous les noms de LIPEX SHEASOFT, LIPEX SHEA-U, LIPEX SHEA, LIPEX SHEALIGHT, LIPEX SHEA TRIS) ou de moringa (moringa oil/hydrogenated moringa oil esters), les cires (acacia decurrens flower wax & helianthus annuus cera seed seed wax, C10-18 triglycerides) , les huiles végétales, le phytosqualane, les alcanes (undecane, tridecane)
   Ledit agent émollient sera présent dans la composition à une teneur de l'ordre de 0.1 à 30%, de préférence 0.5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (commercialisés sous les noms ARISTOFLEX AVC, Aristoflex AVS, Aristoflex HMB, SIMULGEL NS, Simulgel EG, Simulgel 600, Simulgel 800, Pemulen, carbopol, Sepiplus 400, Seppimax zen, Sepiplus S, COSMEDIA SP)
   Ledit agent gélifiant et/ou épaississant sera présent dans la composition à une teneur de l'ordre de 0.1 à 10% en poids total de la composition.
- Un ou plusieurs agent(s) tensioactif(s), tels que les lecithines, les dérivés de polyglycérol, les dérivés de sucre (les dérivés de glucosides ou de xylosides commercialisés sous le nom MONTANOV 68, MONTANOV 202, Montanov 82, MONTANOV L, EASYNOV), les phosphates (C20-22 alkyl phosphate commercialisé sous le nom SENSANOV WR).
   Ledit agent tensioactif, sera présent dans une teneur de l'ordre de 0.1 à 8%, de préférence 0,5 à 3% en poids, par rapport au poids total de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 commercialisés sous le nom NP RIGIN, MATRIXYL 3000, IDEALIFT, EYESERYL), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc.

Ledit agent actif sera présent dans la composition à une teneur de l'ordre de 0.1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

### Utilisation cosmétique non-thérapeutique de l'extrait de Swertia chirata selon l'invention

L'invention a également pour objet l'utilisation cosmétique non thérapeutique d'un extrait de *Swertia chirata* selon l'invention pour prévenir et/ou réduire le vieillissement cutané.

Dans ce mode de réalisation, l'extrait ou la composition est appliqué sur une peau altérée mais non pathologique.

L'invention a encore pour objet l'utilisation cosmétique non thérapeutique d'un extrait de *Swertia chirata* selon l'invention pour hydrater la peau et/ou améliorer la fonction barrière de la peau, comme agent hydratant et/ou apaisant.

L'invention a en outre pour objet l'utilisation cosmétique non thérapeutique d'un extrait de *Swertia chirata* selon l'invention en tant qu'un agent inhibiteur de l'activité des métalloprotéinases matricielles (MMPs).

Enfin, l'invention a également pour objet l'utilisation cosmétique non thérapeutique d'un extrait de *Swertia chirata* selon l'invention en tant qu'un agent stimulateur de l'expression des gènes codant pour les éléments constitutifs de la matrice extracellulaire, notamment stimulant l'expression des gènes codant pour les laminines et les protéoglycanes.

L'invention sera maintenant illustrée par les exemples non limitatifs suivants.

### Brève description des figures

La **Figure 1** est un graphique représentant les voies biologiques modulées par l'extrait de *Swertia chirata* C1 à 0.25% dans des kératinocytes épidermiques humains normaux après 48h de traitement.
La **Figure 2** est un graphique représentant les voies biologiques modulées par l'extrait de *Swertia chirata C2* à 0.25% dans des kératinocytes épidermiques humains normaux après 48h de traitement.

### EXEMPLES :

### Exemple 1 : Préparation et caractérisation des extraits de Swertia chirata.

> L'extrait 1 (extrait «C1 » ou « PFAC1 ») est obtenu au moyen du procédé de préparation suivant :
a) Toutes les parties de *Swertia chirata* préalablement séchées et broyées à une finesse inférieure à 2cm sont extraites 2 fois par macération dans l'éthanol à 60°C pendant 2h avec un rapport solvant/plante de 10/1 ;
b) L'extrait obtenu est ensuite tamisé à 100µm puis filtré jusqu'à 1µm pour éliminer les résidus végétaux ;
b') L'extrait est décoloré avec 30% de charbon actif par rapport à l'extrait sec et ensuite à nouveau filtré à 1µm pour éliminer le charbon résiduel ;
c) De la cellulose est ajoutée à l'extrait pour obtenir un mélange cellulose (40%) / extrait sec (60%) dans l'éthanol ;
d) L'éthanol est ensuite évaporé pour obtenir uniquement le mélange cellulose/extrait sec ;
e) Le mélange cellulose/extrait sec est ensuite broyé à une finesse inférieure à 1cm pour obtenir une poudre ;
f) La poudre obtenue est ensuite extraite par un mélange CO₂ supercritique (95%) / Ethanol (5%) à 60°C, à 500 bars (50Mpa) et avec un débit de 200g/min jusqu'à ce que la poudre ne puisse plus être extraite ;
g) L'extrait obtenu contenu dans l'éthanol est filtré à 1µm ;
h) L'extrait est dilué à 1% dans le palmitate ethyl-2-hexyl puis laissé une nuit à 4°C, et l'éthanol est évaporé ;
i) L'extrait est ensuite filtré à 1µm pour éliminer les insolubles et obtenir l'extrait final.

Après analyse, il a été déterminé que l'extrait 1 comprenait 0.053% en poids de xanthones dont 0.015% de norswertianine, 0.006% de swertianine, 0.002% de bellidine et 0.03% de dérivés d'hydroxyxanthone. L'extrait 1 comprend en outre 0.03% en poids d'acide oléanolic / ursolic (triterpènes). Cet extrait ne comprend pas de swertiamarine détectable.

Cet extrait est un extrait huileux enrichi en molécules apolaires. Il peut donc être avantageusement intégré dans des compositions huileuses/ non aqueuses.

> L'extrait 2 (extrait «C2 » ou « PFAC2 ») est obtenu au moyen du procédé de préparation suivant :
a) Toutes les parties de *Swertia chirata* préalablement séchées et broyées à une finesse inférieure à 2cm sont extraites 2 fois par macération dans l'éthanol à 60°C pendant 2h avec un rapport solvant/plante de 10/1 ;
b) L'extrait obtenu est ensuite tamisé à 100µm puis filtré jusqu'à 1µm pour éliminer les résidus végétaux ;
b') L'extrait est décoloré avec 30% de charbon actif par rapport à l'extrait sec et ensuite à nouveau filtré à 1µm pour éliminer le charbon résiduel ;
c) De la cellulose est ajoutée à l'extrait pour obtenir un mélange cellulose (40%) / extrait sec (60%) dans l'éthanol ;
d) L'éthanol est ensuite évaporé pour obtenir uniquement le mélange cellulose/extrait sec ;
e) Le mélange cellulose/extrait sec est ensuite broyé à une finesse inférieure à 1cm pour obtenir une poudre ;
f) La poudre obtenue est ensuite extraite par un mélange CO₂ supercritique (95%) / Ethanol (5%) à 60°C, à 500 bars (50MPa) et avec un débit de 200g/min jusqu'à ce que la poudre ne puisse plus être extraite ;
g1) Le résidu présent à l'issue de l'étape f) est alors récupéré et remis en solution dans l'éthanol sous agitation à 45°C ;
h1) Le mélange est alors filtré à 1µm pour éliminer la cellulose ;
i1) L'extrait sec est alors dilué à 1% dans le propanediol et l'éthanol est évaporé;
j1) L'extrait est ensuite filtré à 1µm pour éliminer les insolubles et obtenir l'extrait final.

Après analyse, il a été déterminé que l'extrait 2 comprenait 0.134% en poids de xanthones, dont 0.052% de mangiférine, 0.006% de norswertianine, 0.039% de 3-O-demethyleswertipunicoside, 0.001% de bellidine, 0.019% de dixanthone et 0.002% de dérivés d'hydroxyxanthone. L'extrait 2 comprend en outre 0,035% de swertiamarine, 0.282% en poids de sucres/polyols, 0.038% en poids d'acide oléanolic / ursolic (triterpens), 0.024% de minéraux et moins de 0.001% d'amarogentin.

Cet extrait est un extrait hydroalcoolique enrichi en molécules polaires. Il peut donc être avantageusement intégré dans des compositions aqueuses.

### Exemple 2 : Activité des extraits de Swertia chirata C1 et C2

### Protocole :

Des kératinocytes épidermiques humains normaux issus de trois donneurs différents (27 à 34 ans) ont été ensemencés en plaque 6 puits et cultivés en milieu KGM-2 (fourni par la société Lonza) pendant 72heures à 37°C, 5% CO₂. Les cellules ont ensuite été incubées ou non (témoin) avec 0.5% ou 0.25% d'extrait de *Swertia chirata* C1 ou C2 pendant 48hrs. Chaque condition a été effectuée en duplicat. Les ARNs totaux ont été extraits à l'aide du kit RNeasy 96 Plate Extraction Kit (fourni par la société Qiagen) selon les recommandations du fournisseur. La quantité et la qualité des ARNs ont été évaluées par Multiskan GO (fourni par ThermoFischer). Les ADN complémentaires ont été synthétisés et un transcriptome a été réalisé sur puce Affymetrix^{®} GeneChip Human Transcriptome Array 2.0. L'analyse bioinformatique des gènes dont l'expression est modulée par un facteur 2 minimum a été effectuée avec le logiciel Ingenuity Pathway Analysis (IPA^{®}, Qiagen). Ce logiciel recueille des informations sur les interactions molécule à molécule, les réseaux biologiques et les voies canoniques dans la base de données Ingenuity Knowledge.

### Résultats :

► Les extraits de *Swertia chirata* C1 et C2 présentent des profils distincts de régulation génique sur kératinocytes épidermiques

Chacun des deux extraits de *Swertia chirata* module l'expression génique (entre 183 et 636 gènes) dans les kératinocytes épidermiques après 48h de traitement. A la concentration de 0.25%, les extraits régulent 2 à 2,5 fois plus de gènes comparativement à la concentration de 0.5%. Les diagrammes de Venn révèlent que peu de gènes sont communément régulés par les deux extraits avec seulement 35 gènes communs à la concentration de 0.5% et 158 à la concentration de 0.25% (données non fournies).

A l'aide du logiciel IPA^{®}, l'analyse des réseaux biologiques régulés par les deux extraits révèle une cartographie distincte. Ces résultats suggèrent que les extraits de *Swertia chirata* C1 et C2 agissent via des mécanismes biologiques distincts. L'extrait de *Swertia chirata* C1 agit préférentiellement par l'inhibition de voies biologiques tandis que l'extrait de *Swertia chirata* C2 active préférentiellement des voies biologiques dans les kératinocytes.
► L'extrait de *Swertia chirata* C1 inhibe les processus d'inflammation et de dégradation de la matrice extracellulaire médiés par les kératinocytes épidermiques.

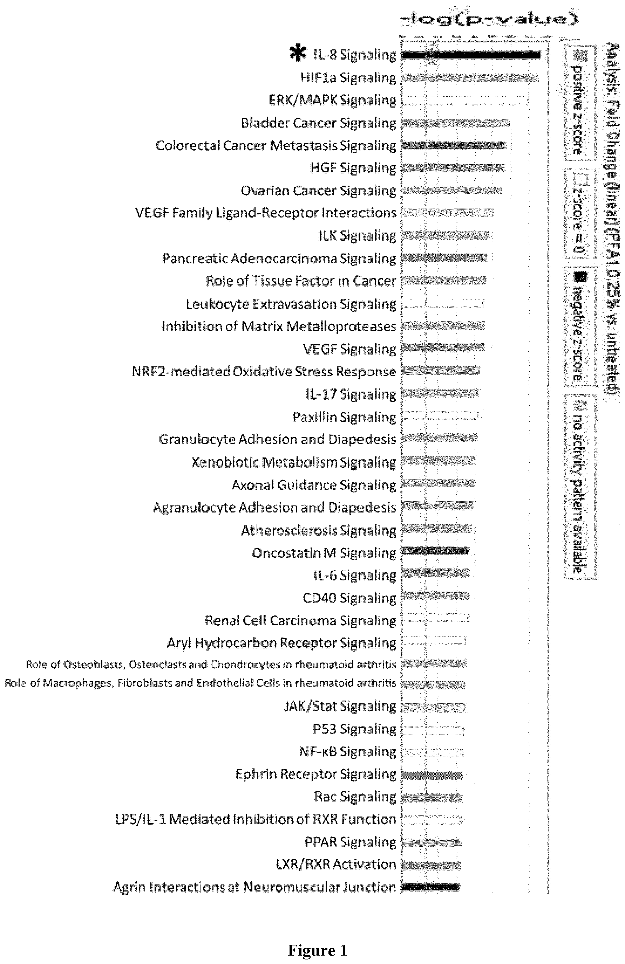

L'analyse des voies biologiques et des gènes modulés par l'extrait de *Swertia chirata* C1 révèle que cet extrait est capable de diminuer la réponse inflammatoire médiée par l'interleukine IL8 (Figure 1, astérisque) et d'inhiber l'expression des métalloprotéinases à l'origine de la dégradation de la matrice extracellulaire, MMP1 (FC=-146), MMP3 (FC=-22), MMP9 (FC=-4) et MMP10 (FC=-21) dans les kératinocytes épidermiques comparativement au témoin non traité. Ces propriétés font que l'extrait de *Swertia chirata* C1 a des rôles anti-inflammatoire et anti-âge cutanés.
► L'extrait de Swertia chirata C2 stimule le métabolisme lipidique des kératinocytes et favorise la synthèse des éléments de la matrice extracellulaire.

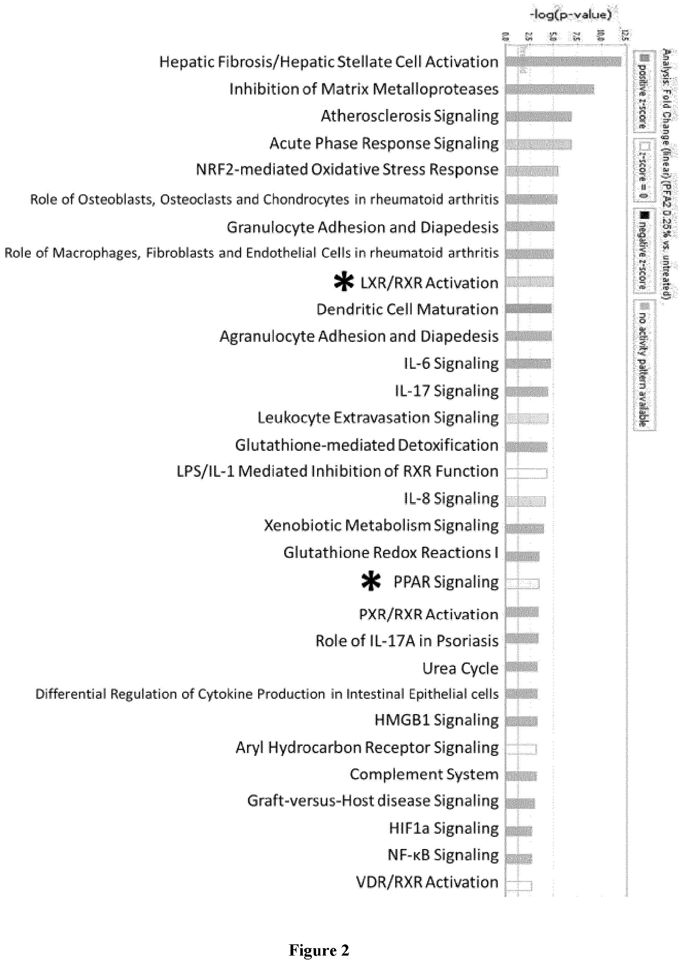

L'analyse des voies biologiques et des gènes modulés par l'extrait de *Swertia chirata* C2 révèle que cet extrait est capable de stimuler des voies biologiques impliquées dans le métabolisme lipidique des kératinocytes épidermiques (voies LXR/RXR et PPAR, Figure 2). De même, l'extrait de *Swertia chirata* 1411PFA C2 est capable de stimuler l'expression de nombreux gènes codant pour des éléments de la matrice extracellulaire (laminines et protéoglycans) ou facteurs activant les fibroblastes (voit Tableau 1 ci-dessous).

**Tableau 1 : Gènes dont l'expression est stimulée par l'extrait de Swertia chirata C2 à 0.25% dans des kératinocytes épidermiques humains normaux après 48h de traitement.**

| Gene | Description | Niveau Expression |
|---|---|---|
| DCN | decorin | 94,74 |
| FN1 | fibronectin 1 | 21,71 |
| FBN1 | fibrillin 1 | 17,29 |
| GPC3 | glypican 3 | 12,42 |
| FBLN5 | fibulin 5 | 10,75 |
| LAMA4 | laminin subunit alpha 4 | 10,46 |
| FAP | fibroblast activation protein alpha | 7,55 |
| FBLN1 | fibulin 1 | 6,93 |
| VIM | vimentin | 6,47 |
| FGF2 | fibroblast growth factor 2 | 5,63 |
| LAMA2 | laminin subunit alpha 2 | 5,52 |
| LUM | lumican | 5,30 |
| LAMC1 | laminin subunit gamma 1 | 4,30 |
| LAMB1 | laminin subunit beta 1 | 3,66 |
| LAMB4 | laminin subunit beta 4 | 2,00 |

Cet extrait a donc des propriétés antiâges, de par son activité stimulatrice de la synthèse de composants de la matrice extracellulaire, mais aussi des propriétés de renforcement de la barrière lipidique cutanée pour maintenir et améliorer l'hydratation de la peau.

**Les extraits de *Swertia chirata* selon l'invention ont une activité antiâge cutanée, médiée par des activités complémentaires, soit par l'inhibition des MMPs, soit par la synthèse d'éléments de la matrice, respectivement.**

### Exemple 2 : Composition cosmétique

Les compositions suivantes peuvent être préparées de façon classique par l'homme du métier. Les quantités indiquées ci-dessous sont exprimées en pourcentages pondéraux. Les ingrédients sont identifiés conformément à la dénomination INCI.

### A - Emulsion huile/eau gel

| **Nom INCI** | **(% W/W)** |
|---|---|
| Limnanthes alba (meadowfoam) seed oil | 1-10 |
| Butyrospermum parkii butter (LIPEX SHEASOFT) | 1-10 |
| Butyrospernum parkii butter extract (LIPEX SHEA TRIS) | 1-10 |
| Camellia oleifera seed oil | 1-10 |
| Caprylic/capric triglycéride | 1-10 |
| Squalane | 1-10 |
| Ammonium acryloyldimethyltaurate/vp copolymer | 0.1-5 |
| Acrylates/c10-30 alkyl acrylate crosspolymer | 0.1-2 |
| Xanthan gum | 0.01-5 |
| Tremella fuciformis (mushroom) extract | 0.01-5 |
| Oryza sativa (rice) powder | 0.1-5 |
| Sodium hyaluronate | 0.01-3 |
| Glycerin | 1-30 |
| Polyquaternium-51 | 1-10 |
| Tocopheryl acetate | 0.1-5 |
| Niacinamide | 0.1-5 |
| Sphingomonas ferment extract | 0.01-5 |
| Polianthes tuberosa | 0.01-5 |
| Betaine | 0.1-10 |
| Sodium pca | 0.5-5 |
| Saccharide isomerate | 0.5-5 |
| Extrait de *Swertia chirata* C1 selon l'invention | 0.001-10 |
| Extrait de *Swertia chirata* C2 selon l'invention | 0.001-10 |
| Yeast extract | 0.1-5 |
| Glycols (Caprylyl Glycol and/or Pentylene Glycol and/or Butylène Glycol and/or propanediol) | 0,1-10 |
| Water | Qs 100 |

### B - Emulsion huile/eau crème

| **Nom INCI** | **(% w/w)** |
|---|---|
| Jojoba esters | 1-5 |
| Limnanthes alba (meadowfoam) seed oil | 0.1-5 |
| Canola oil | 1-10 |
| Argania spinosa kernel oil | 0.1-10 |
| Moringa oil/hydrogenated moringa oil esters | 1-10 |
| C8-12 acid triglycéride | 1-5 |
| Lauroyl lysine | 1-5 |
| Camellia oleifera seed oil | 1-10 |
| Phytosteryl/octyldodecyl lauroyl glutamate | 1-5 |
| Squalane | 1-10 |
| Ammonium acryloyldimethyltaurate/vp copolymer | 1-5 |
| Cetearyl alcohol & cetearyl glucoside | 1-7 |
| Hydrogenated lecithin | 0.1-5 |
| Chondrus crispus (carrageenan) | 0.1-5 |
| Sclerotium gum | 0,01-2 |
| Centella asiatica leaf extract | 0.1-5 |
| Adenosine | 0.1-0.5 |
| Niacinamide | 0.1-5 |
| Secale cereale (rye) seed extract | 0.1-5 |
| Palmitoyl tripeptide-1 & palmitoyl tetrapeptide-7 | 1-5 |
| Plankton extract | 0.1-5 |
| Yeast extract | 1-3 |
| Extrait de *Swertia chirata* C1 selon l'invention | 0.001-10 |
| Extrait de *Swertia chirata* C2 selon l'invention | 0.001-10 |
| Glycyrrhiza glabra extract | 0.001-5 |
| Tranexamic cetyl ester | 0.001-5 |
| Ascorbyl glucoside | 0.001-5 |
| Water | Qs 100 |

### C - Lotion

| **Nom INCI** | **(% w/w)** |
|---|---|
| Glycérine | 1-15 |
| Propanediol | 1-5 |
| Butylène glycol | 1-5 |
| Pentylene glycol | 0.1-5 |
| Caprylyl glycol | 0.01-2 |
| Polyquaternium-51 | 0.1-2 |
| Polianthes tuberosa polysaccharide | 0.1-2 |
| Sodium hyaluronate | 0.01-0.5 |
| Extrait de *Swertia chirata* C2 selon l'invention | 0.001-10 |
| Water | Qs 100 |

### D - Huile

| **Nom INCI** | **(% w/w)** |
|---|---|
| Coco-caprylate/caprate | 5-15 |
| Squalane | 1-15 |
| Camellia oleifera seed oil | 1-20 |
| Helianthus annuus (sunflower) seed oil | 1-20 |
| Simmondsia chinensis (jojoba) seed oil | 1-15 |
| Caprylic/capric triglycéride | 1-15 |
| Limnanthes alba (meadowfoam) seed oil | 1-15 |
| Tocopherol | 0.1-1 |
| Extrait de *Swertia chirata* C1 selon l'invention | 0.001-10 |

Ces compositions peuvent être appliquées tous les jours, matin et/ou soir, sur la peau.

## Revendications

1. Procédé de préparation d'un extrait de *Swertia chirata,* comprenant une étape d'extraction au CO₂ supercritique d'un extrait alcoolique de *Swertia chirata,* à une température comprise entre 40 et 80°C, de préférence entre 50 et 70°C et à une pression comprise entre 20 et 60 MPa, de préférence entre 25 et 35 MPa.

2. Procédé selon la revendication 1, **caractérisé en ce que** l'extraction au CO₂ supercritique est opérée en présence d'un solvant alcoolique, de préférence de l'éthanol.

3. Procédé selon la revendication 2, dans lequel le solvant alcoolique, de préférence l'éthanol, est introduit selon une teneur comprise entre 1 et 10% par rapport au poids total du mélange comprenant le CO₂ supercritique et le solvant alcoolique.

4. Procédé selon l'une quelconque des revendications précédentes, dans lequel un polysaccharide, de préférence de la cellulose, est ajoutée à l'extrait alcoolique avant l'étape d'extraction au CO₂ supercritique.

5. Procédé selon l'une quelconque des revendications précédentes, **caractérisé en ce qu'**il comprend les étapes suivantes :
a) l'extraction de *Swertia chirata,* de préférence sous forme de poudre, avec au moins un solvant alcoolique, de préférence de l'éthanol ;
b) la filtration du mélange obtenu en a) afin d'éliminer les résidus végétaux ;
b') optionnellement, la décoloration du mélange obtenu à l'issue de l'étape b) ;
c) l'ajout d'un polysaccharide, de préférence de la cellulose dans le mélange ainsi obtenu afin d'obtenir un mélange polysaccharide/extrait sec dans le solvant alcoolique ;
d) l'élimination du solvant alcoolique, de préférence par évaporation ;
e) le broyage du mélange extrait sec/polysaccharide obtenu à l'issue de l'étape d) afin d'obtenir une poudre ; et
f) l'extraction au CO₂ supercritique de la poudre obtenue à l'issue de l'étape e) à une température comprise entre 40 et 80°C, de préférence entre 50 et 70°C et à une pression comprise entre 20 et 60 MPa, de préférence entre 25 et 35 MPa.

6. Procédé selon la revendication 5, **caractérisé en ce qu'**il comprend en outre les étapes suivantes :
g) la filtration de l'extrait contenu dans le solvant utilisé pour l'extraction au CO₂ supercritique, et éventuellement l'élimination du solvant alcoolique, de préférence par évaporation ;
h) la dilution de l'extrait obtenu à l'issue de l'étape g) dans une huile, de préférence du palmitate ethyl-2-hexyl, et éventuellement l'élimination du solvant alcoolique résiduel, de préférence par évaporation ;
i) la filtration de l'extrait dilué obtenu à l'issue de l'étape h).

7. Procédé selon la revendication 5, **caractérisé en ce qu'**il comprend en outre les étapes suivantes :
g1) la mise en solution de la poudre résiduelle obtenue à l'issue de l'étape f) dans un solvant alcoolique, de préférence l'éthanol ;
h1) la filtration du mélange obtenu à l'issue de l'étape g1) et éventuellement l'élimination du solvant alcoolique, de préférence par évaporation ;
i1) la dilution de l'extrait obtenu à l'issue de l'étape h1) dans un glycol, de préférence du propanediol, et éventuellement l'élimination du solvant alcoolique résiduel, de préférence par évaporation ;
j1) la filtration de l'extrait dilué obtenu à l'issue de l'étape i1).

8. Extrait de *Swertia chirata,* **caractérisé en ce qu'**il est obtenu au moyen d'un procédé selon l'une quelconque des revendications 1 à 7.

9. Composition cosmétique comprenant, dans un milieu physiologiquement acceptable, au moins un extrait de *Swertia chirata* selon la revendication 8.

10. Utilisation cosmétique non thérapeutique d'un extrait de *Swertia chirata* selon la revendication 8, pour prévenir et/ou réduire le vieillissement cutané.

11. Utilisation cosmétique non thérapeutique d'un extrait de *Swertia chirata* selon la revendication 8, pour hydrater la peau et/ou améliorer la fonction barrière de la peau.

## Patentansprüche

1. Verfahren zum Herstellen eines Extrakts aus Swertia Chirata, umfassend einen Schritt einer Extraktion mit überkritischem CO₂ eines alkoholischen Extrakts aus Swertia Chirata bei einer Temperatur von zwischen 40 und 80°C, vorzugsweise zwischen 50 und 70°C, und bei einem Druck von zwischen 20 und 60 MPa, vorzugsweise zwischen 25 und 35 MPa.

2. Verfahren nach Anspruch 1, **dadurch gekennzeichnet, dass** die Extraktion mit überkritischem CO₂ in Anwesenheit eines alkoholischen Lösungsmittels durchgeführt wird, vorzugsweise von Ethanol.

3. Verfahren nach Anspruch 2, wobei das alkoholische Lösungsmittel, vorzugsweise Ethanol, gemäß einem Gehalt eingegeben wird, welcher zwischen 1 und 10% bezogen auf das Gesamtgewicht der Mischung beträgt, welche das überkritische CO₂ und das alkoholische Lösungsmittel umfasst.

4. Verfahren nach einem der vorhergehenden Ansprüche, wobei ein Polysaccharid, vorzugsweise Zellulose, dem alkoholischen Extrakt vor dem Schritt der Extraktion mit überkritischem CO₂ zugegeben wird.

5. Verfahren nach einem der vorhergehenden Ansprüche, **dadurch gekennzeichnet, dass** es die folgenden Schritte umfasst:
a) Extraktion von Swertia Chirata, vorzugsweise in Pulverform, mit wenigstens einem alkoholischen Lösungsmittel, vorzugsweise Ethanol;
b) Filtration der in a) erhaltenen Mischung, um Pflanzenrückstände zu beseitigen;
b') optional Entfärben der nach Schritt b) erhaltenen Mischung;
c) Zugeben eines Polysaccharids, vorzugsweise Zellulose, zu der derart erhaltenen Mischung, um eine Polysaccharid/Trockenextrakt-Mischung in dem alkoholischen Lösungsmittel zu erhalten;
d) Beseitigen des alkoholischen Lösungsmittels, vorzugsweise durch Verdampfung;
e) Zerkleinern der Trockenextrakt/Polysaccharid-Mischung, welche nach Schritt d) erhalten wird, um ein Pulver zu erhalten; und
f) Extrahieren mit überkritischem CO₂ des Pulvers, welches nach Schritt e) erhalten wird, bei einer Temperatur von zwischen 40 und 80°C, vorzugsweise zwischen 50 und 70°C, und bei einem Druck von zwischen 20 und 60 MPa, vorzugsweise zwischen 25 und 35 MPa.

6. Verfahren nach Anspruch 5, **dadurch gekennzeichnet, dass** es ferner die folgenden Schritte umfasst:
g) Filtration des Extrakts, welches in dem Lösungsmittel enthalten ist, welches für die Extraktion mit überkritischen CO₂ verwendet wird, und ggf. Beseitigen des alkoholischen Lösungsmittels, vorzugsweise durch Verdampfung;
h) Verdünnen des nach Schritt g) erhaltenen Extrakts in einem Öl, vorzugsweise Ethyl-2-Hexyl-Palmitat, und ggf. Beseitigen des verbleibenden alkoholischen Lösungsmittels, vorzugsweise durch Verdampfung;
i) Filtration des verdünnten Extrakts, welcher nach Schritt h) erhalten wird.

7. Verfahren nach Anspruch 5, **dadurch gekennzeichnet, dass** es ferner die folgenden Schritte umfasst:
g1) in Lösung Versetzen des verbleibenden Pulvers, welches nach Schritt f) erhalten wird, in einem alkoholischen Lösungsmittel, vorzugsweise Ethanol;
h1) Filtration der Mischung, welche nach Schritt g1) erhalten wird, und ggf. Beseitigen des alkoholischen Lösungsmittels, vorzugsweise durch Verdampfung;
i1) Verdünnen des Extrakts, welcher nach Schritt h1) erhalten wird in einem Glykol,
vorzugsweise Propandiol, und ggf. Beseitigen des verbleibenden alkoholischen Lösungsmittels, vorzugsweise durch Verdampfen;
j1) Filtration des verdünnten Extrakts, welcher nach Schritt i1) erhalten wird.

8. Extrakt aus Swertia Chirata, **dadurch gekennzeichnet, dass** er mittels eines Verfahrens einem der Ansprüche 1 bis 7 erhalten wird.

9. Kosmetische Zusammensetzung, in einem physiologisch akzeptablen Milieu umfassend wenigstens einen Extrakt aus Swertia Chirata nach Anspruch 8.

10. Nicht-therapeutische kosmetische Verwendung eines Extrakts aus Swertia Chirata nach Anspruch 8 zur Verhinderung und/oder Reduktion der Hautalterung.

11. Nicht-therapeutische kosmetische Verwendung eines Extrakts aus Swertia Chirata nach Anspruch 8 zum Hydrieren der Haut und/oder Verbessern der Barrierefunktion der Haut.

## Claims

1. Method for preparing a *Swertia chirata* extract, comprising a step of extracting, with supercritical CO₂, an alcoholic extract of *Swertia chirata,* at a temperature of between 40 and 80°C, preferably between 50 and 70°C and at a pressure of between 20 and 60 MPa, preferably between 25 and 35 MPa.

2. Method according to Claim 1, **characterized in that** the extraction with supercritical CO₂ is carried out in the presence of an alcoholic solvent, preferably ethanol.

3. Method according to Claim 2, in which the alcoholic solvent, preferably ethanol, is introduced in a content of between 1 and 10% relative to the total weight of the mixture comprising the supercritical CO₂ and the alcoholic solvent.

4. Method according to any one of the preceding claims, in which a polysaccharide, preferably cellulose, is added to the alcoholic extract before the step of extraction with supercritical CO₂.

5. Method according to any one of the preceding claims, **characterized in that** it comprises the following steps:
a) extracting *Swertia chirata,* preferably in powder form, with at least one alcoholic solvent, preferably ethanol;
b) filtering the mixture obtained in a) in order to remove the plant residues;
b') optionally, decolourizing the mixture obtained at the end of step b);
c) adding a polysaccharide, preferably cellulose, to the mixture thus obtained in order to obtain a polysaccharide/dry extract mixture in the alcoholic solvent;
d) removing the alcoholic solvent, preferably by evaporation;
e) milling the dry extract/polysaccharide mixture obtained at the end of step d) in order to obtain a powder; and
f) extracting with supercritical CO₂ the powder obtained at the end of step e) at a temperature between 40 and 80°C, preferably between 50 and 70°C and at a pressure of between 20 and 60 MPa, preferably between 25 and 35 MPa.

6. Method according to Claim 5, **characterized in that** it also comprises the following steps:
g) filtering the extract contained in the solvent used for the extraction with supercritical CO₂, and optionally removing the alcoholic solvent, preferably by evaporation;
h) diluting the extract obtained at the end of step g) in an oil, preferably 2-ethylhexyl palmitate, and optionally removing the residual alcoholic solvent, preferably by evaporation;
i) filtering the diluted extract obtained at the end of step h) .

7. Method according to Claim 5, **characterized in that** it also comprises the following steps:
g1) dissolving the residual powder obtained at the end of step f) in an alcoholic solvent, preferably ethanol;
h1) filtering the mixture obtained at the end of step g1) and optionally removing the alcoholic solvent, preferably by evaporation;
i1) diluting the extract obtained at the end of step h1) in a glycol, preferably propanediol, and optionally removing the residual alcoholic solvent, preferably by evaporation;
j1) filtering the diluted extract obtained at the end of step i1).

8. *Swertia chirata* extract, **characterized in that** it is obtained by the method according to any one of Claims 1 to 7.

9. Cosmetic composition comprising, in a physiologically acceptable medium, at least one *Swertia chirata* extract according to Claim 8.

10. Non-therapeutic cosmetic use of a *Swertia chirata* extract according to Claim 8, for preventing and/or reducing skin ageing.

11. Non-therapeutic cosmetic use of a *Swertia chirata* extract according to Claim 8, for hydrating the skin and/or improving the barrier function of the skin.
